(19) Europäisches Patentamt
European Patent Office
Office européen des brevets

(11) **EP 3 289 956 A1**

(12) **EUROPEAN PATENT APPLICATION**
published in accordance with Art. 153(4) EPC

(43) Date of publication:
**07.03.2018 Bulletin 2018/10**

(51) Int Cl.:
*A61B 1/00* (2006.01)   *A61B 1/04* (2006.01)
*G06T 1/00* (2006.01)   *G06T 7/00* (2017.01)

(21) Application number: **16786375.2**

(22) Date of filing: **20.04.2016**

(86) International application number:
**PCT/JP2016/062486**

(87) International publication number:
**WO 2016/175098 (03.11.2016 Gazette 2016/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.04.2015 JP 2015090620**

(71) Applicant: **Olympus Corporation**
**Hachioji-shi**
**Tokyo 192-8507 (JP)**

(72) Inventors:
• **YAMADA, Tetsuhiro**
  **Hachioji-shi**
  **Tokyo 192-8507 (JP)**
• **YAMANASHI, Momoko**
  **Hachioji-shi**
  **Tokyo 192-8507 (JP)**
• **NAKAMURA Toshio**
  **Hachioji-shi**
  **Tokyo 192-8507 (JP)**
• **TOYAMA, Ryuichi**
  **Hachioji-shi**
  **Tokyo 192-8507 (JP)**

(74) Representative: **Gunzelmann, Rainer**
  **Wuesthoff & Wuesthoff**
  **Patentanwälte PartG mbB**
  **Schweigerstraße 2**
  **81541 München (DE)**

(54) **IMAGE ANALYSIS DEVICE, IMAGE ANALYSIS SYSTEM, AND OPERATION METHOD FOR IMAGE ANALYSIS DEVICE**

(57) An image analysis apparatus (10) includes: an image input section (11); a region extraction section (12) configured to specify a target element including an annular peripheral portion and a center portion that is surrounded by the peripheral portion and that is in a color different from the peripheral portion in a first image and a second image inputted from the image input section (11), the second image being acquired later than the first image, and configured to extract only the center portion of the target element as a region to be analyzed; and a color component extraction section (13) configured to extract respective color component values of the extracted regions to be analyzed of the first and second images.

FIG. 8

**Description**

Technical Field

The present invention relates to an image analysis apparatus, an image analysis system, and an operation method of the image analysis apparatus configured to specify target elements from images of a subject to extract color components.

Background Art

Various image analysis apparatuses configured to specify regions in an image to analyze the image are conventionally proposed.

For example, an electronic endoscope system is described in Japanese Patent Application Laid-Open Publication No. 2012-152266, the electronic endoscope system including: an electronic endoscope configured to photograph inside of a subject; a change region detection section configured to detect, from image data photographed by the electronic endoscope, a change region in which a feature of an image is changed; a mask data generation section configured to generate mask data including parameters of image processing that are set for each pixel such that image processing is applied to the change region and another region in different modes based on the detected change region; and an image processing section configured to apply image processing to the image data based on the mask data.

An image analysis method is described in Japanese Patent Application Laid-Open Publication No. 2007-502185, the image analysis method including: picking up a digital image of dental tissue; determining a first component value of a color of a pixel and a second component value of a color of the pixel for each of a plurality of pixels in the digital image; and calculating a first function value (for example, R/G) of the pixel based on the first component value and the second component value.

An example of an analysis target of the image analysis apparatus includes intestinal villi. Each of the intestinal villi includes a peripheral portion and a center portion in a color different from the peripheral portion. To analyze the image to be analyzed, a user conventionally needs to, for example, find the villi as the analysis target on a screen to extract the villi as focused elements. However, it is difficult to correctly extract a part that changes in the villi to accurately perform quantitative evaluation.

The present invention has been made in view of the circumstances, and an object of the present invention is to provide an image analysis apparatus, an image analysis system, and an operation method of an image analysis apparatus capable of focusing on a feature region that changes in a subject to analyze a color change of the feature region to perform more accurate quantitative evaluation in a necessary region.

Disclosure of Invention

Means for Solving the Problem

An aspect of the present invention provides an image analysis apparatus including: an image input section to which images of a subject acquired over time are inputted; a region extraction section configured to specify a target element including an annular peripheral portion and a center portion that is surrounded by the peripheral portion and that is in a color different from the peripheral portion in each of a first image acquired at a first timing and a second image acquired at a second timing later than the first timing, the first image and the second image being inputted from the image input section, the region extraction section being further configured to extract only the center portion of the target element as a region to be analyzed; and a color component extraction section configured to extract respective color component values of the region to be analyzed of the first image and color component values of the region to be analyzed of the second image extracted by the region extraction section.

An aspect of the present invention provides an image analysis system including: an endoscope inserted into a subject and configured to pick up and acquire images of the subject; and the image analysis apparatus, wherein the images acquired by the endoscope are inputted to the image input section.

An aspect of the present invention provides an operation method of an image analysis apparatus, the operation method including: inputting images of a subject acquired over time to an image input section; a region extraction section specifying a target element including an annular peripheral portion and a center portion that is surrounded by the peripheral portion and that is in a color different from the peripheral portion in each of a first image acquired at a first timing and a second image acquired at a second timing later than the first timing, the first image and the second image being inputted from the image input section, the region extraction section extracting only the center portion of the target element as a region to be analyzed; and a color component extraction section extracting respective color component values of the region to be analyzed of the first image and color component values of the region to be analyzed of the second image extracted by the region extraction section.

second image extracted by the region extraction section.

Brief Description of the Drawings

Fig. 1 is a block diagram showing a configuration of an image analysis system according to a first embodiment of the present invention;

Fig. 2 is a block diagram showing a configuration of a region extraction section according to the first embodiment;

Fig. 3 is a flowchart showing a process using the image analysis system of the first embodiment;

Fig. 4 is a flowchart showing an image analysis process by an image analysis apparatus of the first embodiment;

Fig. 5A is a flowchart showing a process of selecting center portions of a plurality of target elements in a selected region in the image analysis apparatus of the first embodiment;

Fig. 5B is a flowchart showing a modification of the process of selecting center portions of a plurality of target elements in a selected region in the image analysis apparatus of the first embodiment;

Fig. 6 is a flowchart of a double closed curve edge specification process in the image analysis apparatus of the first embodiment;

Fig. 7 is a flowchart showing a single closed curve edge specification process in the image analysis apparatus of the first embodiment;

Fig. 8 is a diagram showing an example of display of images of a subject sorted in chronological order in the first embodiment;

Fig. 9 is a diagram showing a brightness distribution of an image of the subject and an enlarged diagram of one of the target elements in the first embodiment;

Fig. 10 is a diagram showing a structure of intestinal cilia that are the target elements in the first embodiment;

Fig. 11 is a diagram showing an example of regions to be analyzed set in the image of the subject in the first embodiment;

Fig. 12 is a diagram showing an example of a simulation result of brightness of an endoscope in the first embodiment; and

Fig. 13 is a diagram showing an example of a region suitable for extracting color component values obtained from the simulation result of the brightness of the endoscope in the first embodiment.

Best Mode for Carrying Out the Invention

Hereinafter, an embodiment of the present invention will be described with reference to the drawings.

[First Embodiment]

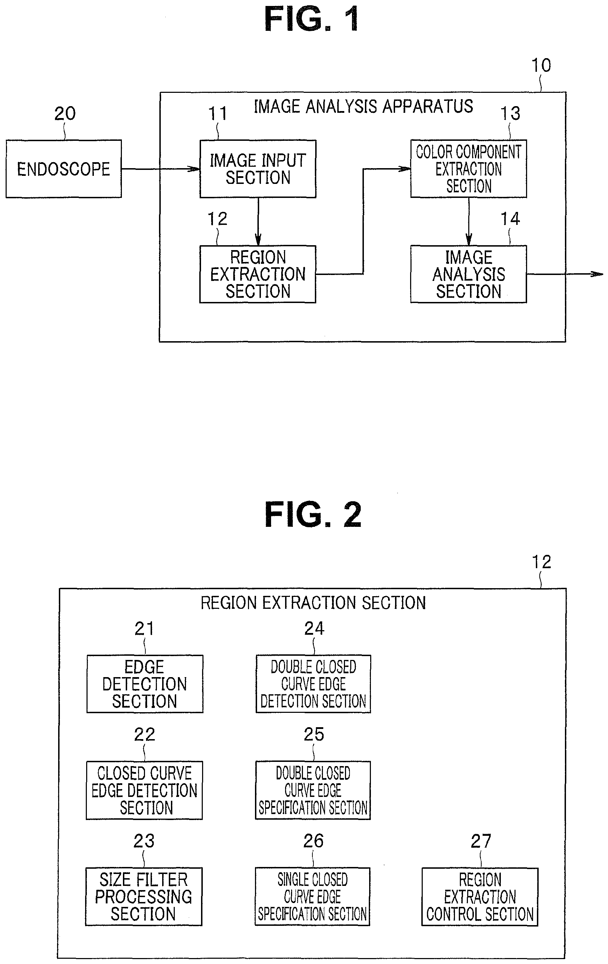

Figs. 1 to 11 show a first embodiment of the present invention, and Fig. 1 is a block diagram showing a configuration of an image analysis system.

The image analysis system includes an endoscope 20 and an image analysis apparatus 10.

The endoscope 20 is inserted into a subject to pick up and acquire an image of a subject. In the present embodiment, the endoscope 20 is capable of, for example, narrow band light observation (NBI: narrow band imaging). Here, to reduce noise components to perform NBI magnified observation, a distal end hood or a distal end attachment is mounted on a distal end of the endoscope 20, for example. In the present embodiment, to apply a load to a subject to observe a change in the subject before and after the load, the endoscope 20 acquires images of the subject over time. To more accurately perceive the change in the subject before and after the application of the load to the subject, it is desirable that setting of brightness of the endoscope 20 is in a same state. Therefore, light adjustment of a light source is not performed before and after the application of the load to the subject, and the images of the subject can be acquired with a constant amount of emitted light from the light source.

The image analysis apparatus 10 includes an image input section 11, a region extraction section 12, a color component extraction section 13, and an image analysis section 14.

The images of the subject acquired by the endoscope 20 over time are inputted to the image input section 11.

The region extraction section 12 specifies target elements, each including an annular peripheral portion and a center portion that is surrounded by the peripheral portion and that is in a color different from the peripheral portion (the target element in the present embodiment is, for example, an image part of intestinal villi that is a feature region as described later), from a first image and a second image inputted from the image input section 11, the first image acquired at a first timing and the second image acquired at a second timing later than the first timing. The region extraction section 12 extracts only the center portions of the target elements as regions to be analyzed.

The color component extraction section 13 extracts color component values of the regions to be analyzed of the first image and color component values of the regions to be analyzed of the second image extracted by the region extraction section 12.

The image analysis section 14 calculates a degree of change between the color component values of the first image and the color component values of the second image extracted from the regions to be analyzed.

Next, Fig. 2 is a block diagram showing a configuration of the region extraction section 12.

The region extraction section 12 is configured to judge a difference between the colors of the peripheral portion and the center portion based on a difference in at least one of hue, saturation, and luminance. Therefore, a difference in color component value indicates a difference in color. For example, when the hue and the saturation are the same and only the luminance is different, the color is different.

As shown in Fig. 2, the region extraction section 12 includes an edge detection section 21, a closed curve edge detection section 22, a size filter processing section 23, a double closed curve edge detection section 24, a double closed curve edge specification section 25, a single closed curve edge specification section 26, and a region extraction control section 27.

The edge detection section 21 applies, for example, an edge detection filter to the images to detect edges.

The closed curve edge detection section 22 further detects edges forming closed curves from the edges detected by the edge detection section 21.

The size filter processing section 23 selects only closed curve edges in which the size is in a possible range of the target elements (for example, possible range of the size of intestinal villi) among the closed curve edges detected by the closed curve edge detection section 22.

The double closed curve edge detection section 24 further detects double closed curve edges with double edges (that is, including an outer closed curve edge and an inner closed curve edge included in the outer closed curve edge) among the closed curve edges detected by the closed curve edge detection section 22 and further selected by, for example, the size filter processing section 23.

The double closed curve edge specification section 25 specifies a region in the inner closed curve edge as a center portion when the color of the region in the inner closed curve edge in the double closed curve edge detected by the double closed curve edge detection section 24 and the color of a region between the inner closed curve edge and the outer closed curve edge are different.

In this case, the double closed curve edge specification section 25 is configured to further specify the region in the inner closed curve edge as a center portion when the color of the region in the inner closed curve edge is in a first color range corresponding to the center portion of the target element (for example, the first color range is a color range close to red when the target element is intestinal villi) and the color of the region between the inner closed curve edge and the outer closed curve edge is in the second color range corresponding to the peripheral portion of the target element (second color range different from the first color range) (for example, the second color range is a color range close to white when the target element is intestinal villi).

Note that the difference in color is judged based on the difference in at least one of the hue, the saturation, and the luminance. Therefore, the color range is a range of one of the hue, the saturation, and the luminance or a range determined by a combination of two or more of the hue, the saturation, and the luminance. For example, the color range may be a range determined by a combination of the hue and the saturation, or the color range may be a luminance range (that is, the center portion and the peripheral portion may be distinguished based only on the luminance). When the target element is intestinal villi and the color range is the luminance range, the first color range can be a range with a lower luminance, and the second color range can be a range with a luminance higher than in the first color range, for example.

It is more preferable that the double closed curve edge specification section 25 specifies the region in the inner closed curve edge as a center portion only when the size filter processing section 23 judges that the sizes of the inner closed curve edge and the outer closed curve edge are in a possible range of the target element.

In the present embodiment, when the number of center portions of the target elements specified by the double closed curve edge specification section 25 is less than a predetermined number (here, the predetermined number is two or more), the single closed curve edge specification section 26 is further used to specify the center portions of the target elements (however, only the single closed curve edge specification section 26 may be used to specify the center portions of the target elements without using the double closed curve edge specification section 25).

The single closed curve edge specification section 26 specifies inside of the region surrounded by the closed curve edge as a center portion when the colors inside and outside of the region surrounded by the closed curve edge detected by the closed curve edge detection section 22 are different.

Note that although the single closed curve edge specification section 26 processes the closed curve edges not subjected to processing by the double closed curve edge specification section 25 in the present embodiment, all of triple, quadruple, ... closed curve edges with more edges than the double closed curve edges are also the double closed curve edges. Therefore, the single closed curve edge specification section 26 processes single closed curve edges.

The single closed curve edge specification section 26 is configured to further specify a region in the single closed curve edge as a center portion when the color of the region in the single closed curve edge is in the first color range corresponding to the center portion of the target element, and the color of a region near the outside of the single closed curve edge is in the second color range corresponding to the peripheral portion of the target element.

It is more preferable that the single closed curve edge specification section 26 specifies the inside of the region surrounded by the single closed curve edge as a center portion only when the size filter processing section 23 judges that the size of the single closed curve edge is in the possible range of the target element.

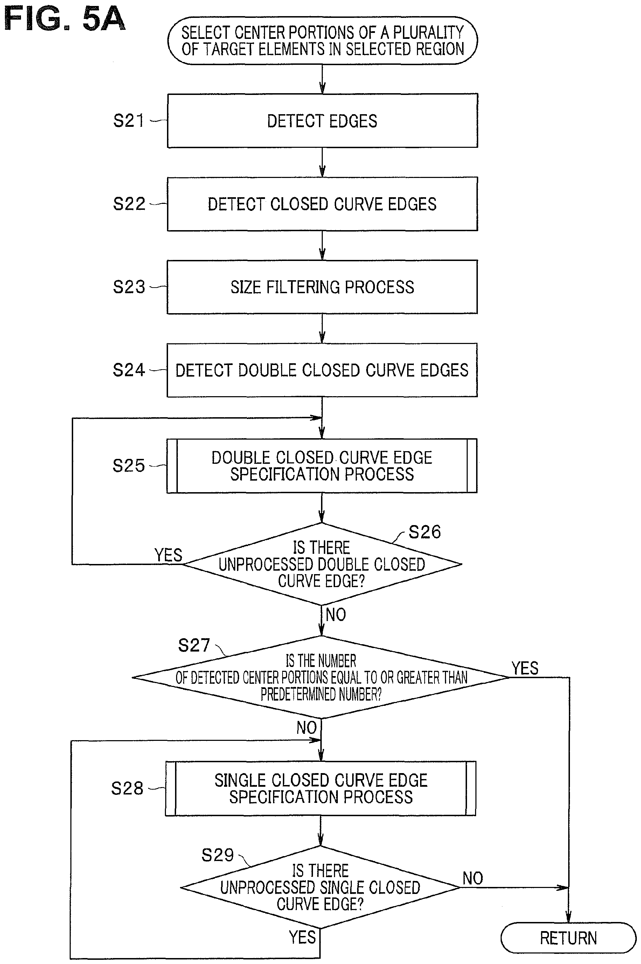

The region extraction control section 27 controls respective sections in the region extraction section 12, that is, the edge detection section 21, the closed curve edge detection section 22, the size filter processing section 23, the double closed curve edge detection section 24, the double closed curve edge specification section 25, the single closed curve edge specification section 26, and the like to cause the sections to perform operation as described later with reference to Figs. 5A to 7.

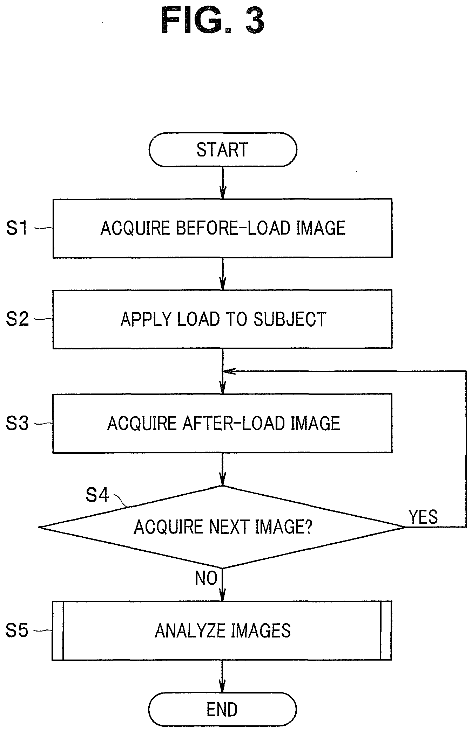

Next, Fig. 3 is a flowchart showing a process using the image analysis system.

When the process is started, the endoscope 20 picks up and acquires an image before the load is applied to the subject (before-load image, first image) at the first timing (step S1). Here, the subject in the present embodiment is, for example, intestinal (more specifically, small intestine) villi (however, the subject is not limited to this, and some other examples include tongue, esophagus, gastric mucosa, and large intestine). At the same time as the acquisition of the image of the subject by the endoscope 20, information of the amount of emitted light at the acquisition of the image may be recorded in, for example, the image analysis apparatus 10 or the endoscope 20.

Subsequently, the load is applied to the subject (step S2). Here, glucose is sprayed as the load, for example (however, the method is not limited to this, and the glucose may be intravenously injected, or other loads may be applied). When the glucose is sprayed, an amount of blood flowing through capillaries increases, and hemoglobin in the blood absorbs more light. Therefore, a part with concentrated capillaries in the villi is observed as a dark part.

Subsequently, the endoscope 20 picks up and acquires an image after the load is applied at a second timing later than the first timing (after-load image, second image) (step S3). When the endoscope 20 acquires the image after the load is applied to the subject, the image is acquired under the same condition as in step S1 with reference to the information of the amount of emitted light if the information of the amount of emitted light is recorded in step S1. Note that a function of deleting the information of the amount of emitted light recorded in step S1 later may be included. The acquisition of the information of the amount of emitted light, the acquisition of the image using the information of the amount of emitted light, and the deletion of the information of the amount of emitted light may be realized by operation of, for example, an operation portion of the endoscope 20, a switch provided on a control panel for controlling the image analysis system, or a foot switch for operating the endoscope 20.

Whether to further acquire a next image is judged (step S4). If it is judged to acquire the next image, the process returns to step S3 to acquire a next after-load image.

If it is judged that the acquisition of the image is finished in step S4, the image analysis apparatus 10 performs image analysis (step S5). The process ends when the image analysis is completed.

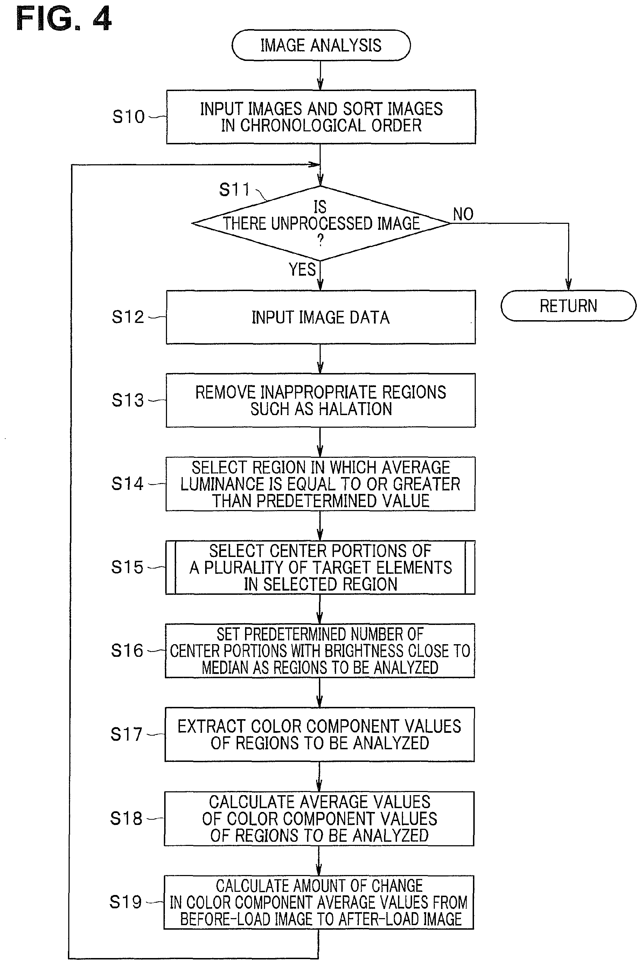

Fig. 4 is a flowchart showing an image analysis process by the image analysis apparatus 10.

When the process is started, the image input section 11 inputs the images of the subject acquired over time from the endoscope 20 and sorts the images in chronological order (step S10).

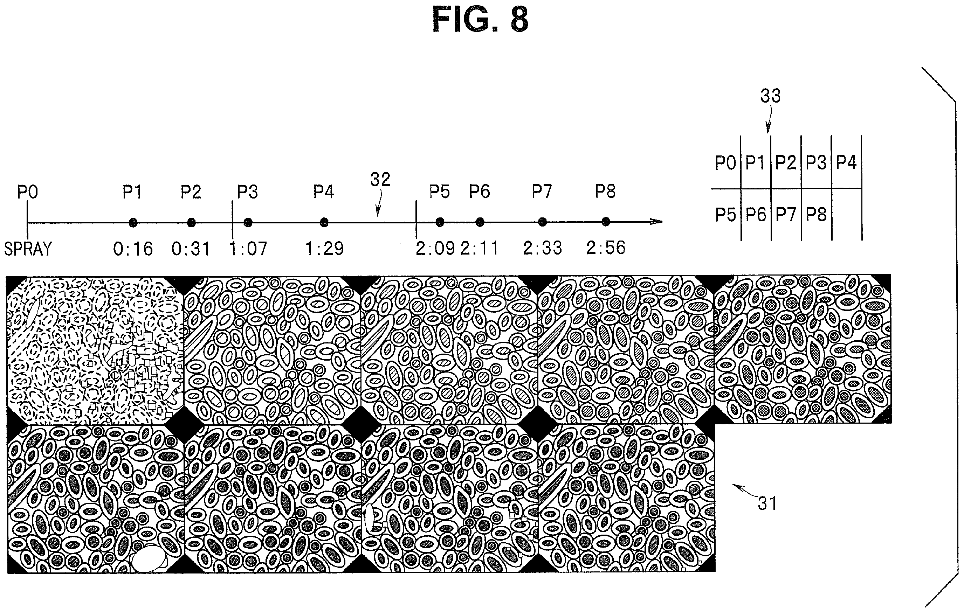

Fig. 8 is a diagram showing an example of display of the images of the subject sorted in chronological order.

In the example of display shown in Fig. 8, an image arrangement display 31, an image acquisition time period display 32, and an image arrangement order display 33 are provided on a display apparatus such as a monitor.

In the image arrangement display 31, acquired images P0 to P8 of the subject are arranged and displayed in order of time period of acquisition.

In the image acquisition time period display 32, time period points of the acquisition of the images P1 to P8 after the application of the load (spray of glucose) are disposed and shown along with, for example, acquisition time periods on a time axis. Note that although the image P0 is an image acquired before the spray of glucose (for example, just before the spray of glucose), the image P0 is displayed at a position of the spray of glucose for the convenience in the example illustrated in Fig. 8 (however, it is obvious that the time axis may be extended to a time point before the spray of glucose to accurately indicate the time point of the acquisition of the image P0).

Furthermore, the image arrangement order display 33 displays the respective images displayed in the image arrangement display 31 in association with the time points of the acquisition of the images P0 to P8 displayed in the image acquisition time period display 32.

Next, the image analysis apparatus 10 judges whether there is an image not yet subjected to a process described later with reference to steps S12 to S19 (step S11).

If it is judged that there is an unprocessed image, the region extraction section 12 inputs image data to be processed from the image input section 11 (step S12).

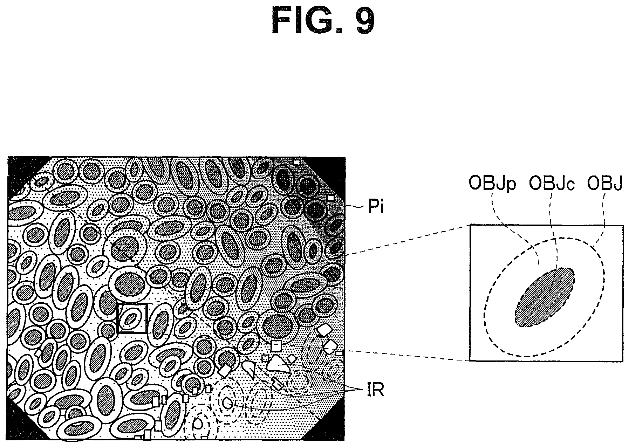

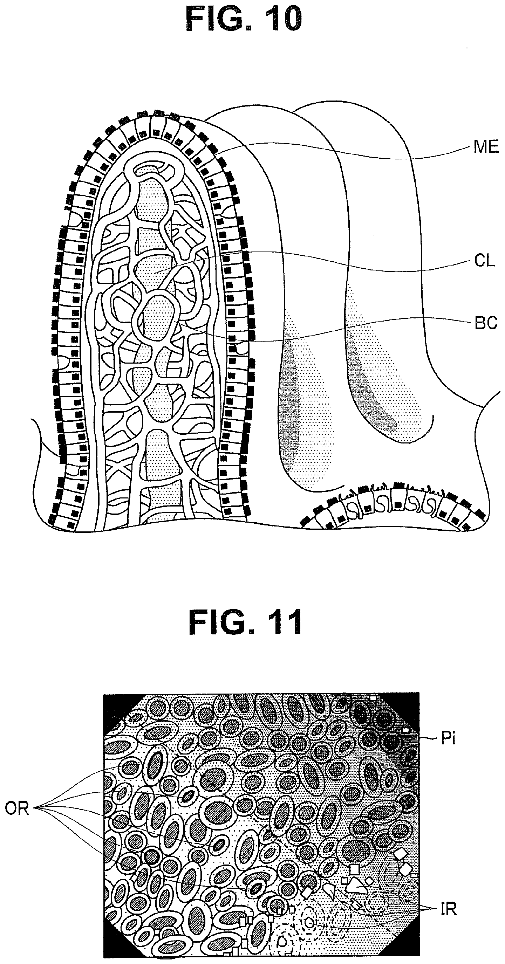

Regions with inappropriate elements (inappropriate regions) IR (see Figs. 9, 11, and the like), such as halation, not suitable for the extraction of color component values are excluded from the processing target (step S13). Other than the regions with halation, examples of the inappropriate regions IR include regions with bubbles and regions out of focus.

Furthermore, a region in which an average luminance calculated for each partial region in a predetermined size in the image is equal to or greater than a predetermined value is selected as an appropriate luminance region (step S14). For example, the average luminance of a region in an upper right half is lower than the predetermined value in an image Pi (here, i is one of 0 to 8 in the example shown in Fig. 8 (that is, Pi is one of P0 to P8)) as shown in Fig. 9 (or Fig. 11). Here, Fig. 9 is a diagram showing a brightness distribution of the image of the subject and an enlarged diagram of one of the target elements.

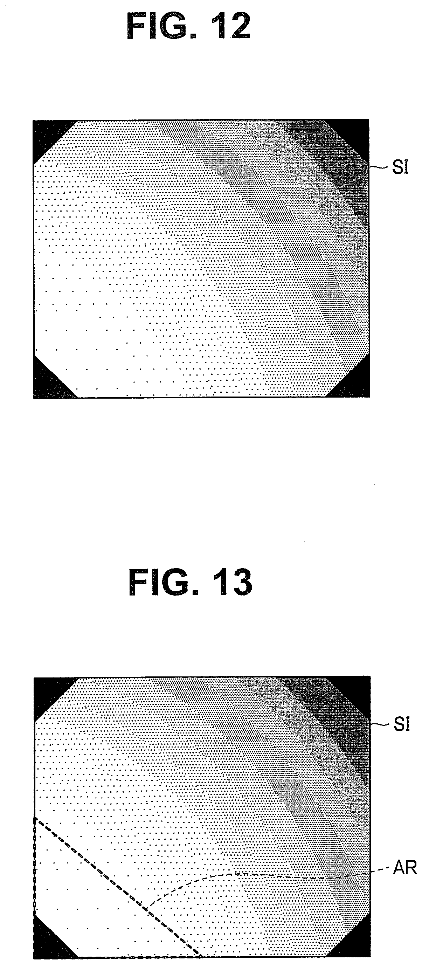

Note that although the region to be analyzed is set by using the image of the subject acquired by the endoscope 20 or the like as an image indicating the performance of the image pickup apparatus configured to acquire the image inputted from the image input section 11 in the description above, the method is not limited to this. A method may also be adopted, wherein a region AR (see Fig. 13) suitable for extracting the color component values from the average luminance calculated for each partial region in the predetermined size is set as the region to be analyzed based on another image indicating the performance of the image pickup apparatus (for example, an image obtained by photographing a flat object with uniform color, such as a test plate and a white balance cap, or an image serving as an index indicating the performance, such as a simulation result SI (see Fig. 12) of brightness obtained from a design value of the endoscope 20). Furthermore, a method may also be adopted, wherein the region to be analyzed is set from the region AR suitable for extracting the color component values based on the average luminance calculated for each partial region in the predetermined size. Here, Fig. 12 is a diagram showing an example of the simulation result SI of the brightness of the endoscope 20, and Fig. 13 is a diagram showing an example of the region AR suitable for extracting the color component values obtained from the simulation result SI of the brightness of the endoscope 20.

Therefore, the region extraction section 12 selects, as an appropriate luminance region, a region in a lower left half of the image Pi in which the average luminance is equal to or greater than the predetermined value. As a result of the selection, a bright region suitable for extracting the color component values is selected, and a dark region not suitable for extracting the color component values is excluded.

Note that although the appropriate luminance range suitable for extracting the color component values is a range in which the average luminance is equal to or greater than the predetermined value here, a region that is too bright in which the average luminance is close to a saturated pixel value may also be excluded. In this case, the appropriate luminance range suitable for extracting the color component values can be a range in which the average luminance is equal to or greater than a predetermined lower limit threshold and equal to or smaller than a predetermined upper limit threshold.

Assuming that the tone of the luminance of the image has, for example, 256 levels of 0 to 255, the lower limit threshold of the appropriate luminance range can be set to, for example, 10 equivalent to a frame part of an endoscopic image, and the upper limit threshold can be set to, for example, 230 equivalent to halation. In this way, the color component of only the object to be analyzed can be extracted, and the accuracy of analysis can be improved.

Subsequently, center portions OBJc (the center portions OBJc are also elements) of a plurality of target elements (image parts of intestinal villi in the present embodiment) OBJ are selected in the selected region (step S15).

As described later with reference to Figs. 5A to 7, image analysis or the like is performed to execute an automatic process to extract and select a plurality of image parts of the intestinal villi that are the target elements OBJ (however, an option for a user to view and manually select the images may be further prepared).

Here, the image part of the intestinal villi that are the target element OBJ is an element including an annular (not limited to a ring shape, and an arbitrary closed curve shape is possible) peripheral portion OBJp and the center portion OBJc that is surrounded by the peripheral portion OBJp and that is in a color different from the peripheral portion OBJp.

Fig. 10 is a diagram showing a structure of the intestinal cilia that are the target elements.

In the intestinal villi, capillaries BC are distributed in a part around a center lymphatic vessel CL at a center portion, and mucosal epithelium ME is formed outside of the capillaries BC to configure the surface of the villi.

When the intestinal villi are magnified and observed by the NBI using light with a narrow-band wavelength that is easily absorbed by hemoglobin in the blood, the part of the capillaries BC is observed in a color different from the mucosal epithelium ME.

When the image part obtained by imaging the villi from above is observed, the image part of the mucosal epithelium ME is observed as the annular peripheral portion OBJp, and the image part of the capillaries BC surrounded by the mucosal epithelium ME is observed as the center portion OBJc with a color different from the mucosal epithelium ME. Therefore, as described later, the difference between the colors of the center portion OBJc and the peripheral portion OBJp is used to determine the target element OBJ.

A predetermined number of (five in an example shown in Fig. 11) center portions OBJc with the brightness close to a median are further selected from a plurality of center portions OBJc selected in this way, and the predetermined number of selected center portions OBJc are set as regions to be analyzed OR (step S16). Here, Fig. 11 is a diagram showing an example of the regions to be analyzed OR set in the image Pi of the subject.

Here, the reason that the center potions OBJc with the brightness close to the median are selected is to analyze portions with brightness most appropriate as samples. Note that a luminance value calculated based on a plurality of color components may be used as the brightness, or a value obtained by simply adding a plurality of color components may be used as an index of the brightness. Other methods may be used to acquire the brightness based on a plurality of color components. In this way, the regions to be analyzed OR set here and shown in Fig. 11 include, for example, five center portions OBJc of the image parts of the intestinal villi.

Next, the color component extraction section 13 extracts color component values, such as an R component value, a G component value, and a B component value, of each pixel included in the regions to be analyzed OR (step S17) and further calculates an average value <R> of the R component values, an average value <G> of the G component values, and an average value <B> of the B component values of the regions to be analyzed OR in the first image (before-load image) and an average value <R'> of the R components values, an average value <G'> of the G component values, and an average value <B'> of the B component values of the regions to be analyzed OR in the second image (after-load image) (step S18).

The image analysis section 14 then calculates an amount of change in color component average values as a degree of change from the before-load image to the after-load image as follows, for example (step S19).

That is, the image analysis section 14 calculates the amount of change as a sum of absolute values of difference values of the color component values between the first image and the second image as shown in the following Equation 1.

[Equation 1]

$$\text{Amount of change} = |\langle R'\rangle\text{-}\langle R\rangle| + |\langle G'\rangle\text{-}\langle G\rangle| + |\langle B'\rangle\text{-}\langle B\rangle|$$

Therefore, the calculated amount of change is a sum of an average value of the color component values in which the values are lower in the second image than in the first image and an average value of the color component values in which the values are higher in the second image than in the first image.

Note that the degree of change from the before-load image to the after-load image calculated by the image analysis section 14 is not limited to the calculation shown in Equation 1.

First, in a first modification, the amount of change as the degree of change is calculated as shown in the following Equation 2, wherein Min (x, y) represents a function for outputting not larger one of x and y (smaller one when x#y).

[Equation 2]

$$\text{Amount of change} = |\text{Min}\,(\langle R'\rangle\text{-}\langle R\rangle,\,0)$$

$$+ \text{Min}\,(\langle G'\rangle\text{-}\langle G\rangle,\,0)$$

$$+ \text{Min}\,(\langle B'\rangle\text{-}\langle B\rangle,\,0)|$$

Therefore, the calculated amount of change is a sum of only the average values of the color component values in which the values are smaller in the second image than in the first image. The calculation method is used in consideration of a characteristic of human eyes. That is, the human eyes more sharply feel a change when the brightness of image changes from bright to dark than when the brightness of image changes from dark to bright. Therefore, the characteristic of human eyes is taken into account such that a change in the image visually perceived by the user coincides with an analysis result of a change in the image obtained by the image analysis.

Next, in a second modification, the amount of change as the degree of change is calculated as shown in the following Equation 3.

[Equation 3]

$$\text{Amount of change} = |\text{Min}\,(\langle R\rangle\text{-}\langle R'\rangle,\,0)$$

$$+ \text{Min}\,(\langle G\rangle\text{-}\langle G'\rangle,\,0)$$

$$+ \text{Min}\,(\langle B\rangle\text{-}\langle B'\rangle,\,0)|$$

Therefore, the calculated amount of change is a sum of only the average values of the color component values in which the values are higher in the second image than in the first image. The reason that the calculation method is used is that a change in the brightness of image from dark to bright is an important analysis result in some cases.

Furthermore, in a third modification, respective color components illustrated on right sides of Equations 1 to 3 are multiplied by weighting factors $\alpha$, $\beta$, and $\gamma$ (here, $\alpha>0$, $\beta>0$, and $\gamma>0$) of respective color components.

For example, in accordance with Equation 1, the amount of change is calculated as shown in the following Equation 4.

[Equation 4]

$$\text{Amount of change} = |\alpha \times <R'>-<R>|$$
$$+ |\beta \times <G'>-<G>|$$
$$+ |\gamma \times <B'>-<B>|$$

Alternatively, in accordance with Equation 2, the amount of change is calculated as shown in the following Equation 5.

[Equation 5]

$$\text{Amount of change} = |\alpha \times \text{Min}(<R'>-<R>, 0)$$
$$+ \beta \times \text{Min}(<G'>-<G>, 0)$$
$$+ \gamma \times \text{Min}(<B'>-<B>, 0)|$$

Alternatively, in accordance with Equation 3, the amount of change is calculated as shown in the following Equation 6.

[Equation 6]

$$\text{Amount of change} = |\alpha \times \text{Min}(<R>-<R'>, 0)$$
$$+ \beta \times \text{Min}(<G>-<G'>, 0)$$
$$+ \gamma \times \text{Min}(<B>-<B'>, 0)|$$

In this case, the weighting factors $\alpha$, $\beta$, and $\gamma$ in Equations 4 to 6 can be adjusted to control how much each color component average value contributes to the amount of change.

In a fourth modification, a rate of change is calculated as the degree of change, in place of the amount of change.

That is, when image pickup conditions (such as exposure time period, aperture value, and illuminance of subject) of each image in a series of image groups (before-load images and after-load images) acquired over time are equal, amounts of change in the image groups, such as a first amount of change from the before-load image P0 to the after-load image P1 and a second amount of change from the before-load image P0 to the after-load image P2, can be compared.

However, the brightness of image generally varies between a plurality of image groups picked up under different image pickup conditions, and the amounts of change cannot be compared as it is in some cases. For example, an amount of change in an image group acquired from a subject and an amount of change in an image group acquired from another subject are compared. If the brightness of one of the image group is twice the brightness of the other image group, the calculated amount of change of one of the image group is twice the calculated amount of change of the other image group even when pathological amounts of change are the same.

Therefore, the rate of change is calculated as the degree of change in the fourth modification to allow the comparison in such a case.

For example, in accordance with Equation 4, the amount of change is calculated as shown in the following Equation 7.

[Equation 7]

$$\text{Amount of change} = \{|\alpha \times <R'> - <R>|$$
$$+ |\beta \times <G'> - <G>|$$
$$+ |\gamma \times <B'> - <B>|\}$$
$$/ |<R> + <G> + <B>|$$

Alternatively, in accordance with Equation 5, the amount of change is calculated as shown in the following Equation 8.

[Equation 8]

$$\text{Amount of change} = |\alpha \times \text{Min}(<R'> - <R>, 0)$$
$$+ \beta \times \text{Min}(<G'> - <G>, 0)$$
$$+ \gamma \times \text{Min}(<B'> - <B>, 0)|$$
$$/ |<R> + <G> + <B>|$$

Alternatively, in accordance with Equation 6, the amount of change is calculated as shown in the following Equation 9.

[Equation 9]

$$\text{Amount of change} = |\alpha \times \text{Min}(<R> - <R'>, 0)$$
$$+ \beta \times \text{Min}(<G> - <G'>, 0)$$
$$+ \gamma \times \text{Min}(<B> - <B'>, 0)|$$
$$/ |<R> + <G> + <B>|$$

Note that Equations 7 to 9 indicate the rates of change corresponding to the amounts of change in Equations 1 to 3 when $\alpha = \beta = \gamma = 1$.

After step S19 is executed, the process returns to step S11 described above. In this way, if it is judged that the processes of all images are executed in step S11, the process returns to a main process not shown.

Fig. 5A is a flowchart showing a process of selecting center portions of a plurality of target elements in the selected region in the image analysis apparatus 10.

When the image analysis apparatus 10 enters the process in step S15 of Fig. 4, the edge detection section 21 applies an edge detection filter to the selected region (for example, region in the lower left half of the image Pi shown in Fig. 9) to extract edge components (step S21).

Next, the closed curve edge detection section 22 further detects edges forming closed curves from the edges detected by the edge detection section 21 (step S22).

Subsequently, the size filter processing section 23 calculates sizes (for example, maximum diameter of closed curve, average diameter, and area of region surrounded by closed curve) of the closed curve edges detected by the closed curve edge detection section 22 and selects only the closed curve edges in which the calculated sizes are in the possible range of the target elements (for example, in the range of the possible size of intestinal villi) (step S23).

The double closed curve edge detection section 24 detects all of the double closed curve edges from the closed curve edges passed through the size filter processing section 23 (step S24).

Note that both the inner closed curve edges and the outer closed curve edges included in the double closed curve edges have passed through the process by the size filter processing section 23 in step S23, and the inner closed curve edges and the outer closed curve edges are closed curve edges judged to have sizes in the possible range of the target elements.

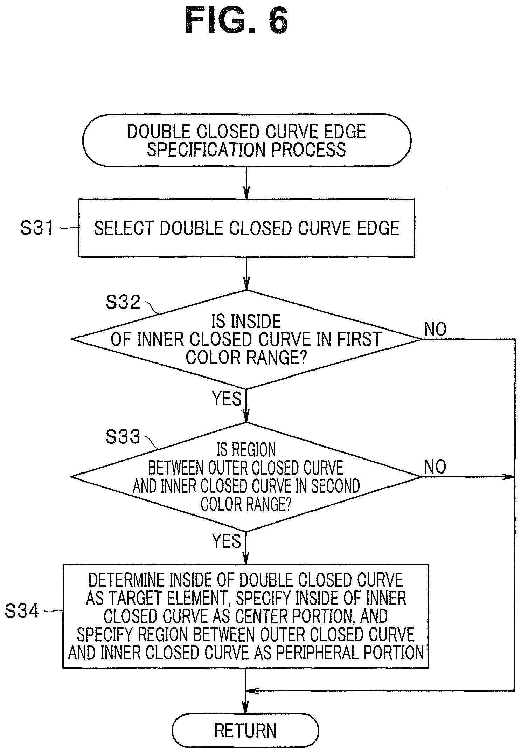

Furthermore, the double closed curve edge specification section 25 executes a process of specifying whether the double closed curve edges detected by the double closed curve edge detection section 24 are the target elements as described later with reference to Fig. 6 (step S25).

Subsequently, the region extraction control section 27 judges whether there is a double closed curve edge not yet subjected to the process of step S25 among the double closed curve edges detected by the double closed curve edge detection section 24 (step S26). If there is a double closed curve edge not yet subjected to the process of step S25, the process of step S25 is applied to a next double closed curve edge.

In this way, if it is judged in step S26 that the process of step S25 is applied to all of the double closed curve edges, the region extraction control section 27 judges whether the number of double closed curve edges judged to be the target elements (further, the number of detected center points of the target elements) is equal to or greater than a predetermined number (five in the example shown in Fig. 11) (step S27).

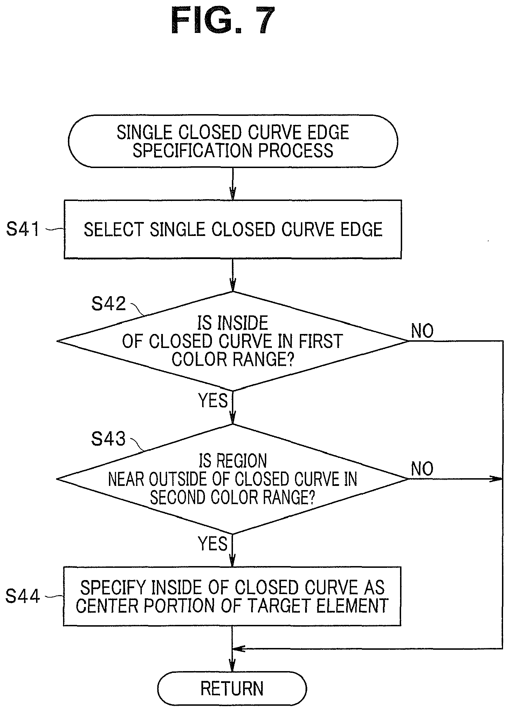

Here, if it is judged that the number of double closed curve edges judged to be the target elements is less than the predetermined number, the single closed curve edge specification section 26 executes a process of specifying whether the single closed curve edges that are not the double closed curve edges (the single closed curve edges are closed curve edges passed through the process by the size filter processing section 23 in step S23 and judged to have sizes in the possible range of the target elements) are the target elements as described later with reference to Fig. 7 (step S28).

Next, the region extraction control section 27 judges whether there is a single closed curve edge not yet subjected to the process of step S25 among the single closed curve edges (step S29). If there is a single closed curve edge not yet subjected to the process of step S25, the process of step S28 is applied to a next single closed curve edge.

In this way, if it is judged in step S29 that the process of step S28 is applied to all of the single closed curve edges or if it is judged in step S27 that the number of double closed curve edges judged to be the target elements is equal to or greater than the predetermined number, the process returns to the process shown in Fig. 4.

In this way, the double closed curve edges that are more likely to be the target elements are first specified, and when the number of double closed curve edges judged to be the target elements is less than the predetermined number, whether the single closed curve edges are the target elements is further specified.

Note that although the single closed curve edges are not specified if the number of double closed curve edges reaches the predetermined number in the process of Fig. 5A, the single closed curve edges may be specified regardless of whether the number of double closed curve edges reaches the predetermined number.

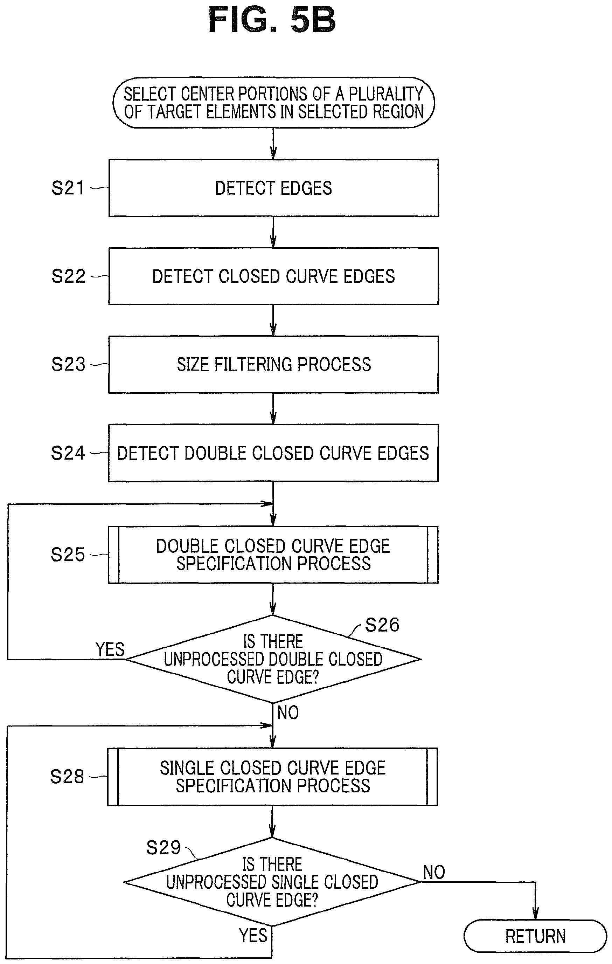

Fig. 5B is a flowchart showing a modification of the process of selecting the center portions of the plurality of target elements in the selected region in the image analysis apparatus.

The process of step S27 in Fig. 5A is eliminated in the process shown in Fig. 5B. As a result, not only the double closed curve edges, but also the single closed curve edges are specified. Therefore, the center portions of more target elements can be selected.

Fig. 6 is a flowchart showing the double closed curve edge specification process in the image analysis apparatus 10.

When the image analysis apparatus 10 enters the process, the double closed curve edge specification section 25 selects one unprocessed double closed curve edge from the double closed curve edges detected by the double closed curve edge detection section 24 in step S24 (step S31).

The double closed curve edge specification section 25 judges whether, for example, the average value of the color component values of the respective pixels inside of the inner closed curve edge of the selected double closed curve edge is in the first color range corresponding to the center portion of the target element (step S32).

Here, if the double closed curve edge specification section 25 judges that the average value is out of the first color range, the double closed curve edge selected in step S31 is not identified as the target element, and the process returns to the process shown in Fig. 5A (or Fig. 5B, the same applies hereinafter, and this will not be repeatedly described).

If the double closed curve edge specification section 25 judges that the average value is in the first color range in step S32, the double closed curve edge specification section 25 further judges whether, for example, the average value of the color component values of respective pixels between the outer closed curve edge and the inner closed curve edge of the selected double closed curve edge is in the second color range corresponding to the peripheral portion of the target element (step S33).

Here, if the double closed curve edge specification section 25 judges that the average value is out of the second color range, the double closed curve edge selected in step S31 is not identified as the target element, and the process returns to the process shown in Fig. 5A.

If the double closed curve edge specification section 25 judges that the average value is in the second color range in step S33 (therefore, if the double closed curve edge specification section 25 judges that the color of the region in the inner closed curve edge and the color of the region between the inner closed curve edge and the outer closed curve edge are different), it is determined that the double closed curve edge selected in step S31 is the target element. The inside of the inner closed curve edge is specified as the center portion of the target element, and the region between the outer closed curve edge and the inner closed curve edge is specified as the peripheral portion of the target element (step S34). The process returns to the process shown in Fig. 5A.

Fig. 7 is a flowchart showing the single closed curve edge specification process in the image analysis apparatus 10.

When the image analysis apparatus 10 enters the process, the single closed curve edge specification section 26 selects one unprocessed closed curve edge in the single closed curve edges other than the double closed curve edges among the closed curve edges passed through the size filter processing section 23 (step S41).

The single closed curve edge specification section 26 then judges whether, for example, the average value of the color component values of the respective pixels inside of the selected single closed curve edge is in the first color range corresponding to the center portion of the target element (step S42).

Here, if the single closed curve edge specification section 26 judges that the average value is out of the first color range, the single closed curve edge selected in step S41 is not identified as the target element, and the process returns to the process shown in Fig. 5A.

If the single closed curve edge specification section 26 judges that the average value is in the first color range in step S42, the single closed curve edge specification section 26 further judges whether, for example, the average value of the color component values of the respective pixels near the outside of the selected single closed curve edge is in the second color range (the second color range different from the first color range) corresponding to the peripheral portion of the target element (step S43).

Here, if the single closed curve edge specification section 26 judges that the average value is out of the second color range, the single closed curve edge selected in step S41 is not identified as the target element, and the process returns to the process shown in Fig. 5A.

If the single closed curve edge specification section 26 judges that the average value is in the second color range in step S43, (therefore, if the single closed curve edge specification section 26 judges that the color of the inside region of the single closed curve edge and the color of the outside near region are different), the inside of the single closed curve edge selected in step S41 is specified as the center portion of the target element (step S44), and the process returns to the process shown in Fig. 5A.

Note that although the respective processes, such as the edge detection (closed curve edge detection, double closed curve edge detection), the size filtering process, and the color range judgement, are executed in Figs. 5A to 7 to increase the detection accuracy of the target elements, any of the processes may be skipped to reduce the processing load to improve the detection speed.

According to the first embodiment, the region extraction section 12 specifies the target element including the annular peripheral portion and the center portion that is surrounded by the peripheral portion and that is in a color different from the peripheral portion and extracts only the center portion of the target element as the region to be analyzed (more specifically, the feature region that changes in the subject is focused to analyze the color change of the feature region). Therefore, more accurate quantitative evaluation can be performed in a necessary region.

The difference between the colors of the peripheral portion and the center portion is judged based on the difference in at least one of the hue, the saturation, and the luminance. Therefore, the judgement can be based on the color component values of the image.

Furthermore, edges are detected from the image, and the edges that form the closed curves are further detected. When the colors inside and outside of the region surrounded by the detected closed curve edge are different, the inside of the region surrounded by the closed curve edge is specified as the center portion. Therefore, the target element including the center portion and the peripheral portion in different colors can be accurately detected.

The inside of the region surrounded by the closed curve edge is specified as the center portion only when the size of the closed curve edge is in the possible range of the target element. Therefore, the detection accuracy of the target element can be further improved.

In addition, the double closed curve edge is further detected, and the region in the inner closed curve edge is specified as the center portion when the color of the region in the inner closed curve edge and the color of the region between the inner closed curve edge and the outer closed curve edge are different. Therefore, the consistency with the shape of the target element including the center portion and the peripheral portion can be higher in the detection.

A plurality of target elements are specified, and the center portions of the plurality of specified target elements are extracted as the regions to be analyzed. Therefore, the color component values of the regions to be analyzed are extracted from more samples, and the degree of change between the color component values of the first image and the color component values of the second image calculated based on the extracted color component values can be a more stable value.

Furthermore, the inappropriate elements not suitable for extracting the color component values are excluded in extracting the regions to be analyzed. Therefore, more accurate image analysis results not affected by the inappropriate elements can be obtained.

The center portions of the predetermined number of target elements in which the brightness is close to the median are extracted as the regions to be analyzed, and the amount of change can be more appropriately obtained.

The regions to be analyzed are extracted from the appropriate luminance regions in the appropriate luminance range in which the average luminance is suitable for extracting the color component values. This can prevent too bright regions and too dark regions, in which the amount of change may not be appropriately reflected on the pixel values even when there is a change in the subject, from becoming the regions to be analyzed.

The advantageous effects can also be attained in images of a subject picked up and acquired by the endoscope 20.

Furthermore, appropriate image analysis can be performed for, for example, intestinal villi.

Note that the respective sections may be configured by circuits. An arbitrary circuit may be implemented as a single circuit as long as the same function can be attained, or the arbitrary circuit may be implemented by combining a plurality of circuits. Furthermore, an arbitrary circuit is not limited to a dedicated circuit for attaining the intended function, and the arbitrary circuit may be configured to cause a general-purpose circuit to execute a processing program to attain the intended function.

Although the image analysis apparatus (or the image analysis system, the same applies hereinafter) is mainly described above, an operation method of causing the image analysis apparatus to operate as described above may be implemented. A processing program for causing a computer to execute a process similar to the image analysis apparatus, a computer-readable non-transitory recording medium recording the processing program, and the like may also be implemented.

Furthermore, the present invention is not limited to the embodiment as it is, and in an execution phase, the constituent elements can be modified without departing from the scope of the present invention to embody the present invention. A plurality of constituent elements disclosed in the embodiment can be appropriately combined to form various aspects of the invention. For example, some of the constituent elements illustrated in the embodiment may be deleted. Furthermore, constituent elements across different embodiments may be appropriately combined. In this way, it is obvious that various modifications and applications can be made without departing from the scope of the invention.

The present application is filed on the basis of claiming the benefit of priority from Japanese Patent Application No. 2015-090620, filed in Japan on April 27, 2015, and the disclosed content is incorporated in the present specification, claims, and drawings by reference.

**Claims**

1. An image analysis apparatus comprising:

   an image input section to which images of a subject acquired over time are inputted;
   a region extraction section configured to specify a target element including an annular peripheral portion and a center portion that is surrounded by the peripheral portion and that is in a color different from the peripheral portion in each of a first image acquired at a first timing and a second image acquired at a second timing later than the first timing, the first image and the second image being inputted from the image input section, the region extraction section being further configured to extract only the center portion of the target element as a region to be analyzed; and
   a color component extraction section configured to extract respective color component values of the region to be analyzed of the first image and color component values of the region to be analyzed of the second image extracted by the region extraction section.

2. The image analysis apparatus according to claim 1, wherein
   the region extraction section judges a difference between colors of the peripheral portion and the center portion based on a difference in at least one of hue, saturation, and luminance.

3. The image analysis apparatus according to claim 2, wherein
   the region extraction section performs edge detection of the images to further detect an edge forming a closed curve, and when colors inside and outside of a region surrounded by the detected closed curve edge are different, the region extraction section specifies the inside of the region surrounded by the closed curve edge as the center portion.

4. The image analysis apparatus according to claim 3, wherein
   the region extraction section further judges whether a size of the closed curve edge is in a possible range of the target element and specifies the inside of the region surrounded by the closed curve edge as the center portion only when the size is in the possible range of the target element.

5. The image analysis apparatus according to claim 3, wherein

the region extraction section further detects a double closed curve edge, and when a color of a region in an inner closed curve edge and a color of a region between the inner closed curve edge and an outer closed curve edge are different in the detected double closed curve edge, the region extraction section specifies the region in the inner closed curve edge as the center portion.

6. The image analysis apparatus according to claim 1, wherein
the region extraction section specifies the target element in plurality and extracts the center portion of each of the plurality of specified target elements as the region to be analyzed.

7. The image analysis apparatus according to claim 6, wherein
the region extraction section extracts the region to be analyzed by excluding an inappropriate region not suitable for extracting color component values.

8. The image analysis apparatus according to claim 6, wherein
the region extraction section extracts, as the region to be analyzed, the center portion of each of a predetermined number of target elements with brightness close to a median among the plurality of specified target elements.

9. The image analysis apparatus according to claim 1, wherein
the region extraction section specifies the target element and extracts the region to be analyzed from an appropriate luminance region in which an average luminance is in an appropriate luminance range suitable for extracting color component values, the average luminance being calculated for each partial region in a predetermined size in an image showing a performance of an image pickup apparatus configured to acquire the images inputted from the image input section.

10. The image analysis apparatus according to claim 1, wherein
the images inputted to the image input section are images picked up and acquired by an endoscope inserted into the subject.

11. The image analysis apparatus according to claim 10, wherein
the target element is an image part of intestinal villi, the center portion is an image part of a region including capillaries in the center portion of the villi, and the peripheral portion is an image part of mucosal epithelium formed on a surface of the villi.

12. An image analysis system comprising:

an endoscope inserted into a subject and configured to pick up and acquire images of the subject; and
the image analysis apparatus according to claim 1, wherein
the images acquired by the endoscope are inputted to the image input section.

13. An operation method of an image analysis apparatus, the operation method comprising:

inputting images of a subject acquired over time to an image input section;
a region extraction section specifying a target element including an annular peripheral portion and a center portion that is surrounded by the peripheral portion and that is in a color different from the peripheral portion in each of a first image acquired at a first timing and a second image acquired at a second timing later than the first timing, the first image and the second image being inputted from the image input section, the region extraction section extracting only the center portion of the target element as a region to be analyzed; and
a color component extraction section extracting respective color component values of the region to be analyzed of the first image and color component values of the region to be analyzed of the second image extracted by the region extraction section.

# FIG. 1

10

IMAGE ANALYSIS APPARATUS

20

```
ENDOSCOPE
```

11

```
IMAGE INPUT
SECTION
```

13

```
COLOR COMPONENT
EXTRACTION
SECTION
```

12

```
REGION
EXTRACTION
SECTION
```

14

```
IMAGE
ANALYSIS
SECTION
```

# FIG. 2

12

REGION EXTRACTION SECTION

21

```
EDGE
DETECTION
SECTION
```

24

```
DOUBLE CLOSED
CURVE EDGE
DETECTION SECTION
```

22

```
CLOSED CURVE
EDGE DETECTION
SECTION
```

25

```
DOUBLE CLOSED
CURVE EDGE
SPECIFICATION SECTION
```

23

```
SIZE FILTER
PROCESSING
SECTION
```

26

```
SINGLE CLOSED
CURVE EDGE
SPECIFICATION SECTION
```

27

```
REGION
EXTRACTION
CONTROL SECTION
```

# FIG. 3

START

S1 — ACQUIRE BEFORE-LOAD IMAGE

S2 — APPLY LOAD TO SUBJECT

S3 — ACQUIRE AFTER-LOAD IMAGE

S4 — ACQUIRE NEXT IMAGE? — YES

NO

S5 — ANALYZE IMAGES

END

# FIG. 4

IMAGE ANALYSIS

S10 — INPUT IMAGES AND SORT IMAGES
IN CHRONOLOGICAL ORDER

S11 — IS
THERE UNPROCESSED IMAGE
?

NO → RETURN

YES

S12 — INPUT IMAGE DATA

S13 — REMOVE INAPPROPRIATE REGIONS
SUCH AS HALATION

S14 — SELECT REGION IN WHICH AVERAGE
LUMINANCE IS EQUAL TO OR GREATER
THAN PREDETERMINED VALUE

S15 — SELECT CENTER PORTIONS OF
A PLURALITY OF TARGET ELEMENTS
IN SELECTED REGION

S16 — SET PREDETERMINED NUMBER OF
CENTER PORTIONS WITH BRIGHTNESS CLOSE TO
MEDIAN AS REGIONS TO BE ANALYZED

S17 — EXTRACT COLOR COMPONENT VALUES
OF REGIONS TO BE ANALYZED

S18 — CALCULATE AVERAGE VALUES
OF COLOR COMPONENT VALUES
OF REGIONS TO BE ANALYZED

S19 — CALCULATE AMOUNT OF CHANGE
IN COLOR COMPONENT AVERAGE VALUES FROM
BEFORE-LOAD IMAGE TO AFTER-LOAD IMAGE

# FIG. 5A

SELECT CENTER PORTIONS OF A PLURALITY
OF TARGET ELEMENTS IN SELECTED REGION

S21 — DETECT EDGES

S22 — DETECT CLOSED CURVE EDGES

S23 — SIZE FILTERING PROCESS

S24 — DETECT DOUBLE CLOSED CURVE EDGES

S25 — DOUBLE CLOSED CURVE EDGE
SPECIFICATION PROCESS

S26 — IS THERE
UNPROCESSED DOUBLE CLOSED
CURVE EDGE?

YES

NO

S27 — IS THE NUMBER
OF DETECTED CENTER PORTIONS EQUAL TO OR GREATER THAN
PREDETERMINED NUMBER?

YES

NO

S28 — SINGLE CLOSED CURVE EDGE
SPECIFICATION PROCESS

S29 — IS THERE
UNPROCESSED SINGLE CLOSED
CURVE EDGE?

NO

YES

RETURN

# FIG. 5B

SELECT CENTER PORTIONS OF A PLURALITY
OF TARGET ELEMENTS IN SELECTED REGION

S21 — DETECT EDGES

S22 — DETECT CLOSED CURVE EDGES

S23 — SIZE FILTERING PROCESS

S24 — DETECT DOUBLE CLOSED CURVE EDGES

S25 — DOUBLE CLOSED CURVE EDGE
SPECIFICATION PROCESS

S26
IS THERE
UNPROCESSED DOUBLE CLOSED
CURVE EDGE?

YES

NO

S28 — SINGLE CLOSED CURVE EDGE
SPECIFICATION PROCESS

S29
IS THERE
UNPROCESSED SINGLE CLOSED
CURVE EDGE?

NO

YES

RETURN

# FIG. 6

```
        ┌─────────────────────────────────┐
        │  DOUBLE CLOSED CURVE EDGE        │
        │    SPECIFICATION PROCESS         │
        └─────────────────────────────────┘
                        │
                        ▼
        ┌─────────────────────────────────┐
  S31 ──│  SELECT DOUBLE CLOSED CURVE EDGE │
        └─────────────────────────────────┘
                        │
                        ▼
  S32             ╱──────────────╲
              ╱─────   IS INSIDE    ─────╲      NO
           ╱  OF INNER CLOSED CURVE IN FIRST ╲───────┐
            ╲        COLOR RANGE?          ╱         │
              ╲─────              ─────╱             │
                    ╲──────────────╱                │
                        │                           │
                       YES                          │
                        ▼                           │
  S33             ╱──────────────╲                  │
              ╱─────  IS REGION    ─────╲           │
         ╱  BETWEEN OUTER CLOSED CURVE     ╲  NO     │
        ╱  AND INNER CLOSED CURVE IN SECOND  ╲───────┤
         ╲          COLOR RANGE?            ╱        │
           ╲─────                    ─────╱          │
                 ╲──────────────────╱               │
                        │                           │
                       YES                          │
                        ▼                           │
        ┌─────────────────────────────────────────┐ │
        │ DETERMINE INSIDE OF DOUBLE CLOSED CURVE  │ │
        │ AS TARGET ELEMENT, SPECIFY INSIDE OF INNER│ │
  S34 ──│ CLOSED CURVE AS CENTER PORTION, AND       │ │
        │ SPECIFY REGION BETWEEN OUTER CLOSED CURVE │ │
        │ AND INNER CLOSED CURVE AS PERIPHERAL PORTION│ │
        └─────────────────────────────────────────┘ │
                        │                           │
                        ▼◄──────────────────────────┘
                ┌───────────────┐
                │     RETURN     │
                └───────────────┘
```

# FIG. 7

```
        ┌─────────────────────────────┐
        │  SINGLE CLOSED CURVE EDGE   │
        │    SPECIFICATION PROCESS    │
        └─────────────────────────────┘
                      │
                      ▼
        ┌─────────────────────────────┐
S41 ──  │ SELECT SINGLE CLOSED CURVE EDGE │
        └─────────────────────────────┘
                      │
                      ▼
S42            ╱IS INSIDE╲                     NO
          ╱OF CLOSED CURVE IN FIRST╲ ──────────────┐
          ╲      COLOR RANGE?      ╱               │
                   ╲      ╱                         │
                  YES │                             │
                      ▼                             │
S43          ╱  IS REGION  ╲                  NO    │
        ╱NEAR OUTSIDE OF CLOSED CURVE IN╲ ─────────►│
        ╲      SECOND COLOR RANGE?      ╱           │
                   ╲      ╱                         │
                  YES │                             │
                      ▼                             │
        ┌─────────────────────────────┐            │
S44 ──  │ SPECIFY INSIDE OF CLOSED CURVE AS │      │
        │ CENTER PORTION OF TARGET ELEMENT │       │
        └─────────────────────────────┘            │
                      │                             │
                      ◄─────────────────────────────┘
                      ▼
              ┌───────────────┐
              │    RETURN     │
              └───────────────┘
```

# FIG. 8

# FIG. 9

# FIG. 10

ME

CL

BC

# FIG. 11

Pi

OR

IR

# FIG. 12

SI

# FIG. 13

SI

AR

## INTERNATIONAL SEARCH REPORT

| International application No. |
| --- |
| PCT/JP2016/062486 |

A. CLASSIFICATION OF SUBJECT MATTER
*A61B1/00*(2006.01)i, *A61B1/04*(2006.01)i, *G06T1/00*(2006.01)i, *G06T7/00*
(2006.01)i

According to International Patent Classification (IPC) or to both national classification and IPC

B. FIELDS SEARCHED

Minimum documentation searched (classification system followed by classification symbols)
A61B1/00, A61B1/04, G06T1/00, G06T7/00

Documentation searched other than minimum documentation to the extent that such documents are included in the fields searched
Jitsuyo Shinan Koho          1922-1996   Jitsuyo Shinan Toroku Koho   1996-2016
Kokai Jitsuyo Shinan Koho    1971-2016   Toroku Jitsuyo Shinan Koho   1994-2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | Michio TANAKA et al., "Tokushu: Plus 30-byo Shikisoho no Katsuyo Shocho Byohen: Shikiso Naishikyo no Katsuyo no Kihon to Kanbetsu Shindan", Endoscopia Digestiva, vol.18, no.12, pages 1869 to 1878, 25 December 2006 (25.12. 2006) | 1-13 |
| A | Michio TANAKA et al., "Shocho Jumo no Keitai Bunrui", Endoscopia Digestiva, vol.26, no.1, pages 75 to 80, 25 January 2014 (25.01.2014) | 1-13 |

☒   Further documents are listed in the continuation of Box C.          ☐   See patent family annex.

| | |
| --- | --- |
| * Special categories of cited documents: | "T"   later document published after the international filing date or priority date and not in conflict with the application but cited to understand the principle or theory underlying the invention |
| "A"   document defining the general state of the art which is not considered    to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| 15 June 2016 (15.06.16) | 28 June 2016 (28.06.16) |

| Name and mailing address of the ISA/ | Authorized officer |
| --- | --- |
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

International application No.

PCT/JP2016/062486

C (Continuation). DOCUMENTS CONSIDERED TO BE RELEVANT

| Category* | Citation of document, with indication, where appropriate, of the relevant passages | Relevant to claim No. |
|---|---|---|
| A | WO 2006/112227 A1 (Olympus Medical Systems Corp.), 26 October 2006 (26.10.2006), entire text; all drawings & JP 2006-288878 A & JP 2006-288879 A & US 2007/0292011 A1 & EP 1870020 A1 & CN 101150977 A & KR 10-2007-0116634 A | 1-13 |
| A | WO 2007/135757 A1 (Olympus Medical Systems Corp.), 29 November 2007 (29.11.2007), entire text; all drawings & US 2007/0276185 A1 & EP 2022386 A1 & CN 101448448 A | 1-13 |

Form PCT/ISA/210 (continuation of second sheet) (January 2015)

**EP 3 289 956 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012152266 A
- JP 2007502185 A
- JP 2015090620 A